# EUROPEAN PATENT APPLICATION

(11) **EP 4 356 902 A1**
(43) Date of publication of application: **24.04.2024**
(21) Application number: 23204440.4
(22) Date of filing: 18.10.2023
(51) Int. Cl.: A61K 9/20, A61K 9/28, A61K 9/48, A61K 31/4439

(54) **A CAPSULE COMPRISING LANSOPRAZOLE**

(30) Priority: 19.10.2022 TR 202215876
(71) Applicant: SANOVEL ILAC SANAYI VE TICARET A.S., Istanbul 34460 (TR)
(72) Inventor: SUNEL, Fatih, Istanbul (TR); BILGEHAN ATAK, Fadime, Istanbul (TR); ATAMAN, Seval, Istanbul (TR); YAZICI, Ozlem, Istanbul (TR); SARP, Onder, Istanbul (TR)
(74) Representative: Sevinç, Erkan

(57) **Abstract**

The present invention relates to a pharmaceutical composition in the form of capsule having micro or mini tablet wherein; the tablets comprising a core tablet comprising lansoprazole and an enteric coating comprising at least one enteric coating polymer. The capsules are obtained by a process which is simple, rapid, cost effective, time-saving and industrially convenient process.

## Description

### Field of the Invention

The present invention relates to a pharmaceutical composition in the form of capsule having micro or mini tablet wherein; the tablets comprising a core tablet comprising lansoprazole and an enteric coating comprising at least one enteric coating polymer. The capsules are obtained by a process which is simple, rapid, cost effective, time-saving and industrially convenient process.

### Background of the Invention

Lansoprazole, chemical name 2-(((3-methyl-4-(2,2,2-trifluoroethoxy)-2-pyridyl)methyl) sulfinyl)-1H-benzoyl Imidazole, brownish white crystalline powder, easily soluble in dimethylamide, soluble in methanol, insoluble in ethanol and ether, almost insoluble in water. It has a chemical structure which is shown in the Formula I.

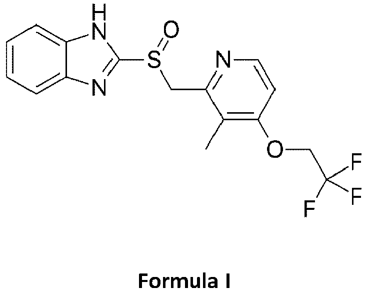

Lansoprazole is the second proton pump inhibitor anti-ulcer drug developed by Takeda Corporation of Japan after omeprazole. 1992ln 1999, Japan's Takeda Pharmaceutical Co., Ltd. and Houde Company first officially put it on the market in France, and in May 1995, it was approved by the FDA to be listed in the United States.

Lansoprazole is extremely unstable under acidic conditions, and is degraded to inactive components in a short time under low pH environment, so it is easily degraded in gastric acid environment. In order to improve the bioavailability of the drug, it needs to be prepared as an enteric-coated preparation.

Chinese Patent Application No.: 201010245689.6 discloses a lansoprazole enteric capsule formulation and preparation method, using sodium bicarbonate as a stabilizer, cross-linked polyvinylpyrrolidone as a disintegrant, and acrylic resin as an enteric coating agent.

U.S. Patent No. 6,328,994 discloses an orally disintegrable lansoprazole tablet which comprises fine enteric coated granules having an average particle diameter of 400µπ 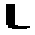 or less.

Therefore, it is still necessary to prepare a pharmaceutical composition in the form of capsule having micro or mini tablet that the desired stability, the desired dissolution profile and is suitable for industrialized production.

### Detailed Description of the Invention

The main object of the present invention is to eliminate problems caused by active substance and bringing additional advantages to the relevant prior art. To explain in more detail, the main purpose is to provide a pharmaceutical composition in the form of capsule having enteric coating micro or mini tablets having high physical and chemical stability and a long shelf life by the help of selection of excipients.

Another object of the present invention is to obtain a pharmaceutical composition in the form of capsule having enteric coating micro or mini tablets providing improved dissolution profile, it also is cost effective.

Another object of the present invention is to obtain a pharmaceutical composition in the form of capsule having enteric coating micro or mini tablets providing improved excellent pharmacomechanic properties, such as flowability, compressibility and content uniformity.

In particular, there is a need to combine an acid-labile physiologically active substance, with basic inorganic salts and so forth for stability, and further to coat with coating layers such as an enteric layer. It is an important problem to produce small enteric coated micro or mini tablets, even though it contains the acid-labile physiologically active substance in small content. In this invention, the weight ratio of lansoprazole to disintegrant is 1.0-5.0. So, the composition provides the desired profile and flowability, and compressibility. Also, to provide the desired stability, pH modifiers are used in the present invention.

According to one embodiment of the present invention, the tablets in the capsule can be microtablets or minitablets, this varies according to the size of the tablets. The tablets in capsule are cylindrical with a flat or convex upper side and lower side and with a diameter and height which are preferably approximately equal and, independently of one another, preferably have a tablet size of from about 1 mm to about 4 mm, more preferably from about 1.5 mm to about 4.5 mm. The tablets in capsule have a tablet weight of about 1 mg to about 50 mg.

According to one embodiment of the present invention, a pharmaceutical composition in the form of capsule having micro or mini tablet wherein; the tablets comprising
- core tablet comprising lansoprazole and at least one pH modifier, at least one disintegrant,
- enteric coating comprising at least one enteric coating polymer, at least one plasticizer and at least one anti-sticking agent,

Wherein the weight ratio of lansoprazole to disintegrant is 1.0-5.0.

According to one embodiment of the present invention, the amount of lansoprazole is between 5.0 % and 20.0% by weight in the total composition.

According to one embodiment of the present invention, the amount of core tablet is between 60.0 % and 85.0% by weight in the total composition. Preferably, it is between 65.0 % and 80.0% by weight in the total composition.

According to one embodiment of the present invention, the core tablet comprises lansoprazole, further comprises at least one diluent or at least one binder or at least one lubricant or at least one glidant or at least one surfactant or mixtures thereof.

Suitable disintegrants are selected from the group comprising croscarmellose sodium, low substituted hydroxypropyl cellulose, crospovidone, ion-exchange resins, magnesium aluminium silica, sodium carboxymethyl cellulose, calcium silicate, sodium starch glycolate, carboxymethyl cellulose, calcium carboxymethyl cellulose, docusate sodium, polyacryline potassium, poloxamer, povidone, sodium alginate, sodium glycine carbonate, or mixtures thereof.

According to one embodiment of the present invention, the disintegrant is croscarmellose sodium or crospovidone mixtures thereof.

According to one embodiment of the present invention, the amount of disintegrant is between 1.0 % and 15.0% by weight in the total composition.

According to one embodiment of the present invention, the disintegrants are present in the core tablet.

According to one embodiment of the present invention, the weight ratio of lansoprazole to disintegrant is 1.3-4.0.

Suitable pH modifiers are selected from the group comprising magnesium carbonate, aluminum potassium sulfate, anhydrous citric acid, anhydrous disodium hydrogen phosphate, potassium carbonate, anhydrous sodium dihydrogen phosphate, dibasic potassium sulfate, calcium carbonate, dilute hydrochloric acid, monobasic potassium phosphate, phosphoric acid, adipic acid, sodium acetate, sodium bicarbonate, sodium carbonate, sodium citrate, sodium dihydrogen phosphate dihydrate, tribasic sodium phosphate or mixtures thereof. Using a Ph modifier ensures that lansoprazole, which is unstable at low pH, remains at the desired stability under long conditions.

According to one embodiment of the present invention, the pH modifier is sodium bicarbonate or magnesium carbonate or mixtures thereof.

According to one embodiment of the present invention, the pH modifier is present in core tablet or the enteric coating or both.

Suitable plasticizers are selected from the group comprising triethyl citrate, polyethylene glycol, triacetin, alpha tocopherol, docusate sodium, glyceryl monooleate, glyceryl monostearate, tributyl citrate, acetyl triethyl citrate, acetyl tributyl citrate, butyl stearate, Stearyl alcohol, propylene glycol, diethyl phthalate, dioctyl phosphate, sodium lauryl sulphate, sorbitan esters, potassium cetylphosphate, ethylene glycol distearate, sodium dodecanoate, dioctyl sodium sulfosuccinate, sodium stearate, benzalkonium chlorides, polysorbates, poloxamers or mixtures thereof.

According to one embodiment of the present invention, the plasticizer is triethyl citrate in the enteric coating.

Suitable diluents are selected from the group comprising D-mannitol, lactose, microcrystalline cellulose, corn starch, lactose monohydrate, spray-dried mannitol, lactose, starch, dextrose, sucrose, fructose, maltose, sorbitol, xylitol, inorganic salts, calcium salts, polysaccharides, dicalcium phosphate, sodium chloride, maltodextrin, sucrose-maltodextrin mixture, trehalose, magnesium carbonate or mixtures thereof.

According to one embodiment of the present invention, the diluent is lactose or microcrystalline cellulose or corn starch or mixtures thereof.

According to one embodiment of the present invention, the amount of diluents is between 20.0 % and 70.0% by weight in the total composition. The diluents and the using amount provide the desired content uniformity and bulk volume for compressibility.. Since the amount of the active ingredient is low compared to the whole composition, it is important to ensure content uniformity.

According to one embodiment of the present invention, the diluents are present in the core tablet.

Suitable binders are selected from the group comprising hydroxypropyl cellulose, polyvinylpyrrolidone, natural gums, gelatin, alginates, sodium alginate, gum, guar gum, starch mucilage, acacia mucilage, cetostearyl alcohol, pullulan, polydextrose, polyethylene oxide, pectin, aluminum hydroxide, hyaluronic acid or mixtures thereof.

According to one embodiment of the present invention, the binder is hydroxypropyl cellulose or polyvinylpyrrolidone or mixtures thereof.

According to one embodiment of the present invention, the binders are present in the core tablet.

According to one embodiment of the present invention, the amount of binder is between 0.1 % and 10.0% by weight in the total composition. The amount provides the desired granule quality, content uniformity, and release profile. Since the amount of active ingredient is low compared to the whole composition, it is important to ensure granule quality and homogeneity while taking into consideration the release profile.

Suitable glidants are selected from the group comprising talc, colloidal silica anhydrous, colloidal silicon dioxide, calcium silicate, aluminum silicate, magnesium silicate, magnesium oxide, starch or mixtures thereof.

According to one embodiment of the present invention, the glidant is talc or colloidal silica anhydrous or colloidal silicon dioxide or mixtures thereof. Using these glidants provides the flowability.

According to one embodiment of the present invention, when talc is used in the core tablet, it acts as a glidant.

According to one embodiment of the present invention, the glidants are present in the core tablet or the enteric coating.

According to one embodiment of the present invention, the amount of glidant is between 0.1 % and 8.0% by weight in the total composition.

Suitable surfactants are selected from the group comprising polysorbate 80, glyceryl monostearate, sodium dodecyl sulphate, sodium lauryl sulfate, cetylpyridinium chloride, docusate sodium, lauric acid, polyoxyethylene sorbitan fatty acid esters (polysorbate), phospholipids, cetrimide or mixtures thereof.

According to one embodiment of the present invention, the surfactant is polysorbate 80 or sodium dodecyl sulphate or sodium lauryl sulfate or mixtures thereof. It is a synthetic organic compound widely used in pharmaceutical products as an anionic surfactant. In the present invention, it provides the desired profile. It also helps to provide flowability.

According to one embodiment of the present invention, the surfactants are present in the core tablet or the enteric coating or both.

Suitable anti-sticking agents are selected from the group comprising talc, starch, kaolin, lactose or dextrose or mixtures thereof.

According to one embodiment of the present invention, the anti-sticking agent is talc.

According to one embodiment of the present invention, the amount of anti-sticking agents is between 0.005 % and 1.0% by weight in the total composition.

According to one embodiment of the present invention, the anti-sticking agents are present in the enteric coating or both.

According to one embodiment of the present invention, the enteric coating helps to protect lansoprazole from acidic degradation while the dosage form transits the stomach. The enteric coating comprises at least one enteric coating polymer or at least one anti-sticking agent or at least one plasticizer or at least one surfactant or at least one pH modifiers or mixtures thereof.

Suitable enteric coating polymers are selected from the group comprising methacrylic acid-methyl methacrylate copolymer (1:2)(Eudragit S100), methacrylic acid-methyl methacrylate copolymer (1:1)(Eudragit L100), hydroxyl propyl methylcellulose (HPMC), glyceryl behenate, hydroxyethyl methylcellulose, hydroxypropyl cellulose, hydroxypropyl ethylcellulose, ethylcellulose, methacrylic acid - ethyl acrylate copolymer, polymethylmetacrylate, polyvinyl acetate, polyvinyl alcohol, polyvinylpyrrolidine, glyceryl dibehenate, polyethylene oxide, polyethylene glycol, cellulose acetate, vinyl acetate/crotonic acid copolymers, maleic anhydride/methyl vinyl ether copolymers, copolymer of acrylic or methacrylic acid esters, polyoxyethylene -alkyl ethers or mixtures thereof. It is used only in the enteric coating.

According to one embodiment of the present invention, the enteric coating polymer is methacrylic acid-methyl methacrylate copolymer (1:2) (Eudragit S100) or methacrylic acid-methyl methacrylate copolymer (1:1)(Eudragit L100) or methacrylic acid - ethyl acrylate copolymer (Eudragit L 30 D-55 or Kollicoat MAE 30DP) or mixtures thereof. The methacrylic acid copolymer is used alone or in combination with other enteric polymers to achieve the desired delayed release profile. It also helps to provide stability.

Suitable lubricants are selected from the group comprising magnesium stearate, sodium stearyl fumarate, calcium stearate, stearic acid, polyethylene glycol, sodium lauryl sulfate, magnesium lauryl sulfate, fumaric acid, glyceryl palmito sulfate, hydrogenated vegetable oil, zinc stearate or mixtures thereof.

According to one embodiment of the present invention, the lubricant is magnesium stearate.

According to one embodiment of the present invention, the lubricant is present in the core tablet.

According to one embodiment of the present invention, the amount of lubricant is between 0.1 % and 5.0% by weight in the total composition.

According to one embodiment of the present invention, the core tablet comprises lansoprazole, magnesium stearate, microcrystalline cellulose, magnesium carbonate.

According to one embodiment of the present invention, the enteric coating comprises methacrylic acid ethyl acrylate copolymer (1.1), talc, triethyl citrate.

### Example 1:

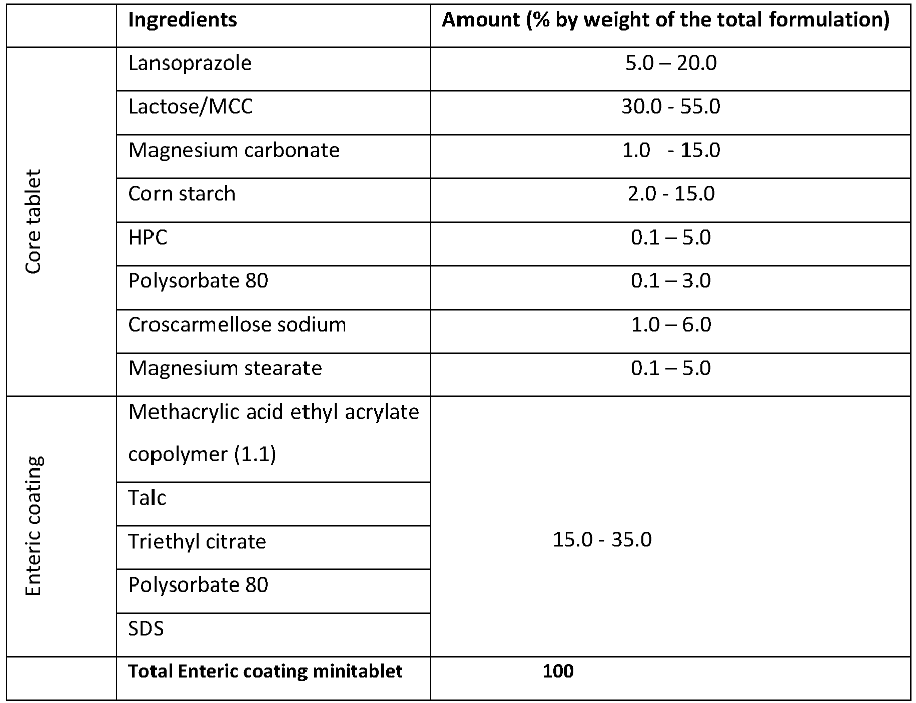

A process for example 1;
Core tablet
   - Weighing 0.85 sieved Lansoprazole, magnesium carbonate, lactose or MCC, ½ croscarmellose sodium, and mixing for 15 minutes,
   - Granulating the dry mixture with HPC and polysorbate 80 in water solution,
   - Sieving 2.0 mm wet granules,
   - Drying the granules in oven,
   - Sieving dry granules into 0.85mm,
   - Adding ½ croscarmellose sodium, and mixing for 5 minutes,
   - Adding weighed and 0.6 mm sieved Magnesium stearate to dry mixture and mix for 3 minutes,
   - Compressing the mixture into the tablet,
Enteric coating
   - Performing enteric coating with Methacrylic acid copolymer dispersion,
Filling the micro or mini tablets having inner and enteric coating into capsules.

### Example 2:

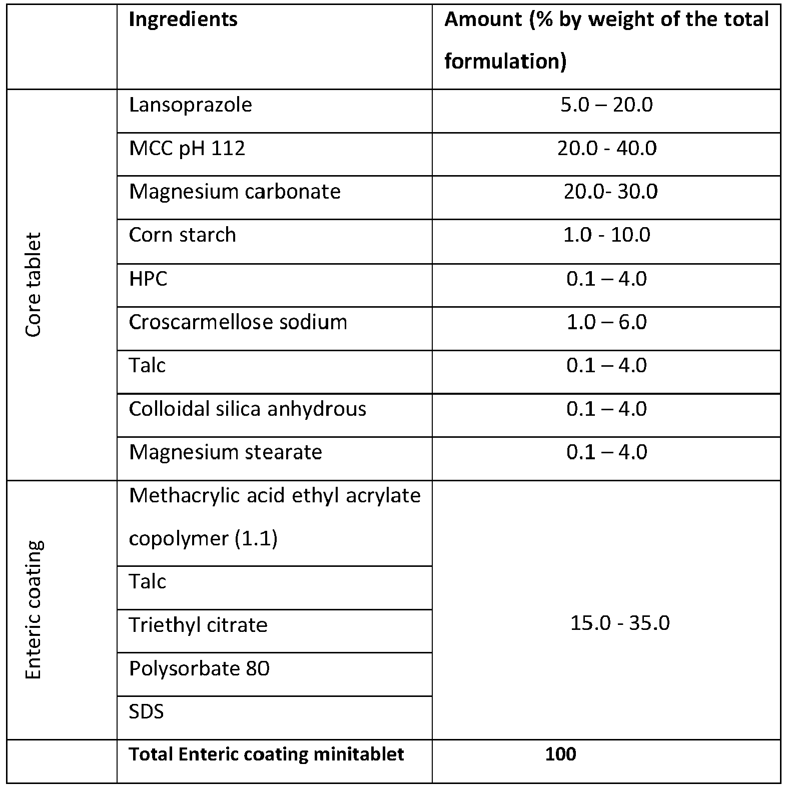

A process for example 2;
Core tablet
   - Weighing Lansoprazole, Magnesium carbonate, MCC 112, HPC, corn starch, croscarmellose sodium, colloidal silica anhydrous, talc and mixing for 15 minutes,
   - Adding weighed and 0.6 mm sieved Magnesium stearate to dry mixture and mix for 3 minutes,
   - Compressing the mixture into the tablet,
Enteric coating
   - Performing enteric coating with Methacrylic acid copolymer dispersion,
Filling the micro or mini tablets having enteric coating into capsules.

### Example 3:

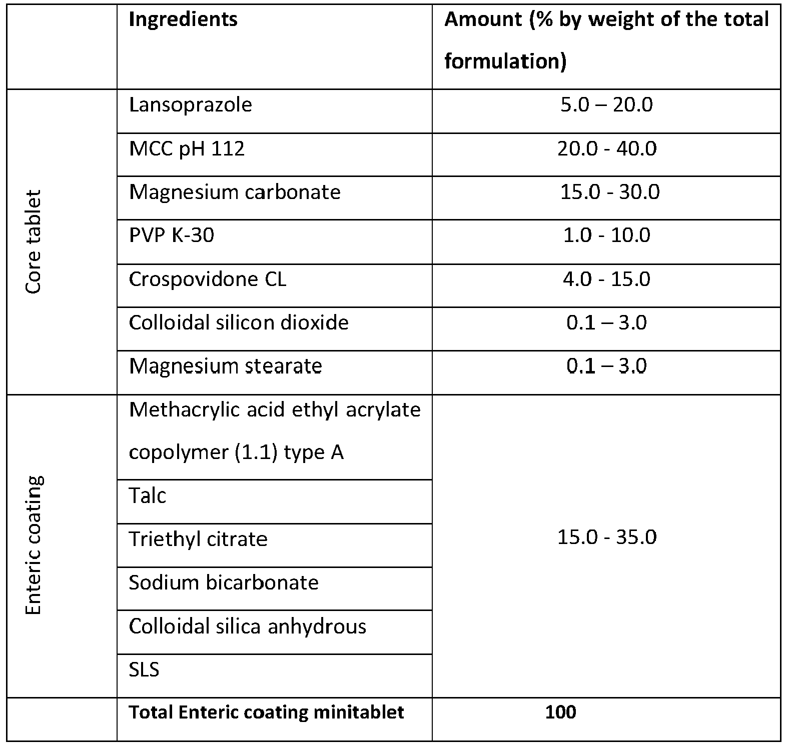

A process for example 3;
Core tablet
   - Weighing Lansoprazole, Magnesium carbonate, ½ MCC 112, colloidal silicon dioxide, PVP K-30, and mixing for 10 minutes,
   - Adding weighed and 0.6 mm sieved ½ Magnesium stearate to dry mixture and mix for 3 minutes,
   - Performing slug granulation in compactor,
   - Sieving dry granules 1.2 m,
   - Adding ½ MCC, crospovidone CL, and mixing for 5 minutes,
   - Adding 0.6 mm sieved ½ magnesium stearate, and mixing for 3 minutes,
   - Compressing the mixture into the tablet,
Enteric coating
   - Performing enteric coating with Methacrylic acid copolymer dispersion,
Filling the micro or mini tablets having inner and enteric coating into capsules.

## Claims

1. A pharmaceutical composition in the form of capsule having micro or mini tablet wherein;
the tablets comprising;
- core tablet comprising lansoprazole and at least one pH modifier, at least one disintegrant,
- enteric coating comprising at least one enteric coating polymer, at least one plasticizer and at least one anti-sticking agent,
Wherein the weight ratio of lansoprazole to disintegrant is 1.0-5.0.

2. A pharmaceutical composition according to claim 1, wherein the core tablet comprises lansoprazole, further comprises at least one diluent or at least one binder or at least one lubricant or at least one glidant or at least one surfactant or mixtures thereof.

3. A pharmaceutical composition according to claim 1, wherein disintegrants are selected from the group comprising croscarmellose sodium, low substituted hydroxypropyl cellulose, crospovidone, ion-exchange resins, magnesium aluminum silica, sodium carboxymethyl cellulose, calcium silicate, sodium starch glycolate, carboxymethyl cellulose, calcium carboxymethyl cellulose, docusate sodium, polyacryline potassium, poloxamer, povidone, sodium alginate, sodium glycine carbonate, or mixtures thereof.

4. A pharmaceutical composition according to claim 3, wherein the disintegrant is croscarmellose sodium or crospovidone mixtures thereof.

5. A pharmaceutical composition according to claim 1, wherein pH modifiers are selected from the group comprising magnesium carbonate, aluminum potassium sulfate, anhydrous citric acid, anhydrous disodium hydrogen phosphate, potassium carbonate, anhydrous sodium dihydrogen phosphate, dibasic potassium sulfate, calcium carbonate, dilute hydrochloric acid, monobasic potassium phosphate, phosphoric acid, adipic acid, sodium acetate, sodium bicarbonate, sodium carbonate, sodium citrate, sodium dihydrogen phosphate dihydrate, tribasic sodium phosphate or mixtures thereof.

6. A pharmaceutical composition according to claim 5, wherein the pH modifier is sodium bicarbonate or magnesium carbonate or mixtures thereof.

7. A pharmaceutical composition according to claim 1, wherein plasticizers are selected from the group comprising triethyl citrate, polyethylene glycol, triacetin, alpha tocopherol, docusate sodium, glyceryl monooleate, glyceryl monostearate, tributyl citrate, acetyl triethyl citrate, acetyl tributyl citrate, butyl stearate, Stearyl alcohol, propylene glycol, diethyl phthalate, dioctyl phosphate, sodium lauryl sulphate, sorbitan esters, potassium cetylphosphate, ethylene glycol distearate, sodium dodecanoate, dioctyl sodium sulfosuccinate, sodium stearate, benzalkonium chlorides, polysorbates, poloxamers or mixtures thereof.

8. A pharmaceutical composition according to claim 7, wherein the plasticizer is triethyl citrate in the enteric coating.

9. A pharmaceutical composition according to claim 2, wherein diluents are selected from the group comprising D-mannitol, lactose, microcrystalline cellulose, corn starch, lactose monohydrate, spray-dried mannitol, lactose, starch, dextrose, sucrose, fructose, maltose, sorbitol, xylitol, inorganic salts, calcium salts, polysaccharides, dicalcium phosphate, sodium chloride, maltodextrin, sucrose-maltodextrin mixture, trehalose, magnesium carbonate or mixtures thereof.

10. A pharmaceutical composition according to claim 2, wherein binders are selected from the group comprising hydroxypropyl cellulose, polyvinylpyrrolidone, natural gums, gelatin, alginates, sodium alginate, gum, guar gum, starch mucilage, acacia mucilage, cetostearyl alcohol, pullulan, polydextrose, polyethylene oxide, pectin, aluminum hydroxide, hyaluronic acid or mixtures thereof.

11. A pharmaceutical composition according to claim 10, wherein the amount of binder is between 0.1 % and 10.0% by weight in the total composition.

12. A pharmaceutical composition according to claim 1, wherein enteric coating further comprises at least one surfactant or at least one pH modifiers or mixtures thereof.

13. A pharmaceutical composition according to claim 1, wherein the core tablet comprises lansoprazole, magnesium stearate, microcrystalline cellulose, magnesium carbonate.

14. A pharmaceutical composition according to claim 1, wherein the enteric coating comprises methacrylic acid ethyl acrylate copolymer (1.1), talc, triethyl citrate.
